# EUROPÄISCHE PATENTANMELDUNG

(11) **EP 4 462 377 A1**
(43) Veröffentlichungstag der Anmeldung: **13.11.2024**
(21) Anmeldenummer: 24171995.4
(22) Anmeldetag: 23.04.2024
(51) Int. Cl.: G06V 10/25, A61B 1/00, G06V 10/26, G06V 10/44, G06V 20/20

(54) **VERFAHREN UND VORRICHTUNG ZUR ÜBERBLENDUNG VON OBJEKTEN IN DIGITALEN BILDERN**

(30) Priorität: 03.05.2023 DE 102023111479
(71) Anmelder: Karl Storz SE & Co. KG, 78532 Tuttlingen (DE)
(72) Erfinder: Buschle, Lukas, 78532 Tuttlingen (DE); Steinke, Bernd, 78532 Tuttlingen (DE); Haag, Simon, 78532 Tuttlingen (DE); Jasmin, Keuser, 78532 Tuttlingen (DE)

(57) **Zusammenfassung**

Die Erfindung betrifft ein Verfahren zur zumindest teilweisen optischen Überlagerung von mindestens einer Information im Vordergrund eines mit einer medizinischen Bildgebungsvorrichtung aufgenommenen digitalen Bildes von einem Betrachtungsbereich mit Bildinformationen, die dem, im Blickfeld der medizinischen Bildgebungsvorrichtung, durch die wenigstens eine Information verdecktem Bereich entspricht.

## Beschreibung

### Technisches Gebiet

Die vorliegende Erfindung betrifft ein Verfahren sowie eine Vorrichtung zum Überblenden von Objekten im Vordergrund digitaler Bilder mit, dem jeweiligen Hintergrund entsprechenden, Bildinformationen.

### Hintergrund

In der Endoskopie werden Endoskope verwendet, um den Arbeitsbereich im Inneren eines Patienten zu visualisieren und ein oder mehrere endoskopische Instrumente, um den entsprechenden medizinischen Eingriff durchzuführen. Hierbei werden die Instrumente in der Regel in einem sehr spitzen Winkel relativ zum Endoskop eingeführt, wodurch der Instrumentenschaft sehr nahe am Endoskop ist und entsprechend sehr groß im Bild zu sehen ist.

Gegenstände, wie endoskopische Instrumente, die nahe an dem Endoskop liegen, nehmen einen großen Teil vom Bild ein, was dazu führen kann, dass der Benutzer, wie ein operierender Arzt, weniger des eigentlichen Arbeitsbereichs sehen kann und gegebenenfalls wichtige Bereiche von dem Instrument verdeckt werden. In speziellen Anwendungsfällen, wie zum Beispiel der Cystoscopy, reduziert sich der Winkel zwischen Instrument und Endoskop auf 0 Grad; hier werden Instrument und Endoskop auf der gleichen Achse bewegt, wodurch in der Visualisierung die Überlagerung des Arbeitsbereichs durch das Instrument noch massiver ausfallen kann.

Daneben können Instrumente oder Teile von Instrumenten, wie Instrumentenschäfte, die nahe an der Optik und damit auch im Wirkungsbereich der Lichtquelle liegen, Licht reflektieren und somit zu Reflektionen im Bild führen. Diese Reflektionen können den Gesamteindruck des Bildes verschlechtern, indem zum Beispiel Helligkeit im Hintergrund reduziert wird oder der Kontrast durch Streulicht reduziert wird.

Entsprechend ist es eine Aufgabe der vorliegenden Erfindung die Einschränkungen und Defizite der aus dem Stand der Technik bekannten Verfahren und Systeme, wenigstens teilweise, zu überwinden. Es ist insbesondere eine Aufgabe der vorliegenden Erfindung geeignete Verfahren und Systeme zur Verfügung zu stellen, die es erlauben, bestimmte Instrumente, Teile von Instrumenten und/oder andere, gegebenenfalls störende oder irrelevante Objekte oder Informationen im endoskopischen Blickfeld zu erkennen und mit Bildinformation des Hintergrundes des Bildes zu überlagern und so quasi transparent darzustellen, so dass der Benutzer die relevanten Strukturen oder Informationen im Hintergrund erkennen kann. Gleichzeitig sollte für Benutzer des Verfahrens oder der Systeme erkennbar sein, welche Informationen aktuell und präzise dem realen endoskopischen Bild entsprechen und welche etwas verzögert oder im Detail unsicherer sind, aber z.B. die Orientierung im Arbeitsbereich erlauben.

### Zusammenfassung der Erfindung

Entsprechend offenbart die vorliegende Erfindung Verfahren zur wenigstens teilweisen optischen Überlagerung von wenigstens einer Information im Vordergrund eines digitalen Bildes, insbesondere eines endoskopischen Bildes. Das erfindungsgemäße Verfahren umfasst hierbei das Aufnehmen mit einer medizinischen Bildgebungsvorrichtung, wenigstens eines digitalen Bildes in einem Blickfeld der medizinischen Bildgebungsvorrichtung, das Segmentieren, durch eine Bildbearbeitungsvorrichtung, wenigstens eines Bildbereichs des aufgenommenen wenigstens einen Bildes, das Auswählen, durch die Bildverarbeitungsvorrichtung, des wenigstens einem Bildbereichs in dem wenigstens einem digitalen Bild, welcher der wenigstens einen Information entspricht, das Überlagern, durch die Bildbearbeitungsvorrichtung, des Bildbereichs in dem wenigstens einem Bild mit Bildinformation und das Darstellen, auf einer Darstellungsvorrichtung, des wenigstens einen Bildes zusammen mit dem überlagerten Bildbereich. Das erfindungsgemäße Verfahren ist dadurch gekennzeichnet, dass die Bildinformation welche für die Überlagerung verwendet wird, dem, im Blickfeld der medizinischen Bildgebungsvorrichtung durch die wenigstens eine Information verdeckten Bereich entspricht. In Ausführungsformen der vorliegenden Erfindung ist die medizinische Bildgebungsvorrichtung eine Endoskop Kamera, insbesondere eine Stereoendoskopkamera, oder eine Exoskopkamera.

Eine Information im Sinne der vorliegenden Erfindung kann jede Art von bildlichen Elementen in einem Bild sein. Informationen können insbesondere Abbildungen von Gegenständen, wie Instrumente oder Teile von Instrumenten, aber auch optische Effekte wie Reflexionen oder Überblendungen einzelner Pixel oder Gruppen von Pixeln. So sind insbesondere auch gesättigte d.h. maximal weiße Bereiche, z.B. durch Reflektionen, überstrahlte Randbereiche, durch Laser überblendete einzelne Frames oder Bereiche einzelner Frames oder auch bei Rolling Shutter basierten Sensoren mit Laser überblendete einzelne Zeilen und Teil-Zeilen Informationen im Sinne der vorliegenden Erfindung.

Segmentieren in Sinne der vorliegenden Erfindung ist das Erfassen und Erzeugen inhaltlich zusammenhängende Regionen durch Zusammenfassung benachbarter Pixel entsprechend bestimmter Homogenitätskriteria. Grundsätzlich kann die Segmentierung im Rahmen der vorliegenden Erfindung auf verschiedene Weise, insbesondere mittels pixel-, kanten- und regionenorientierter Verfahren erfolgen. Zusätzlich kann zwischen modellbasierten Verfahren, bei denen von einer bestimmten Form der Objekte ausgegangen wird, und texturbasierten Verfahren, bei denen auch eine innere homogene Struktur der Objekte berücksichtigt wird, unterschieden werden. Grundsätzlich können alle der vorgenannten Verfahren, auch in Kombination, im Rahmen der vorliegenden Erfindung Anwendung finden. Insbesondere kann die Segmentierung auch mit Hilfe künstlicher Intelligenzalgorithmen erfolgen. Hierbei ist es insbesondere vorteilhaft, wenn der Algorithmus mit Gegenständen trainiert wurde, die in den Bildern der nachfolgenden Anwendung der Verfahren enthalten sein können. So ist es bei der Verwendung von Endoskopen bei medizinischen Eingriffen vorteilhaft, wenn der Algorithmus die verschiedenen Instrumente, die in einem medizinischen Eingriff verwendet werden, kennt, um so die Qualität der Segmentierung zu verbessern. Diese Erkennung kann gegebenenfalls manuell, halb-automatisch oder automatisch erfolgen und kann gegebenenfalls auch unter Verwendung künstlicher Intelligenzalgorithmen erfolgen. Gegebenenfalls können die Segmentierung und die Erkennung gleichzeitig erfolgen. Insbesondere können sogenannte Mehrklassen oder multiclass Segmentierungsalgorithmen verwendet werden, bei denen die segmentierten Bildbereiche bestimmen Klassen zugeordnete werden. Im Beispiel des endoskopischen, medizinischen Eingriffs können beispielsweise die Klassen Gewebe, Arbeitselement (also der Werkzeugbereich eines endoskopischen Instruments) und Instrumenten Schaft verwendet werden. Somit können segmentierte Bildbereiche gleich zugeordnet und erkannt werden und basierend auf dieser Zuordnung können durch das erfindungsgemäße Verfahren bestimmte Bildbereiche, wie zum Beispiel der Instrumentenschaft, überlagert und transparent dargestellt werden.

In Ausführungsformen des erfindungsgemäßen Verfahrens kann die Bildinformation für der Überlagerung perspektivisch an den Bildbereich angepasst werden, auf dem die Überlagerung erfolgen soll. Hierfür können insbesondere Strukturen, Kontraste und Farbbereiche erfasst und in Übereinstimmung gebracht werden.

In Ausführungsformen des erfindungsgemäßen Verfahrens kann die Bildinformation aus wenigstens einem Bild extrahiert werden, dass zeitlich vor dem wenigstens ein digitales Bild aufgenommen wurde und in dem die Bildinformation, die dem in dem wenigstens einem digitalen Bild durch die wenigstens eine Information verdecktem Bereich entspricht, enthalten ist.

In einigen Ausführungsformen kann die Bildinformation mit Hilfe von auf künstlicher Intelligenz basierenden generativen Bildverarbeitungsmethoden durch die Bildverarbeitungsvorrichtung erzeugt werden. Hierbei können mittels auf künstliche Intelligenz basierender Algorithmen, wie beispielsweise InPainting oder OutPainting, optisch passende, künstliche Bildinformationen generiert werden. Diese Verfahren werden insbesondere dann ausgeführt, wenn für den zu überlagernden Bildbereich, die keine adäquaten Bildinformationen verfügbar sind. Diese Verfahren basieren auf einem sukzessiven Fortführen der Randinformationen in fehlenden Bereich, z.B. das Bildinnere, hinein, um so die durch die Verdeckung des Gegenstandes entstandene Lücke in der Darstellung zu füllen. Die angewandten Algorithmen verlängern beispielsweise Linien gleicher Grauwerte am Rand iterativ in den zu füllenden Bereich hinein. Anschließend könne Farben in den zu füllenden Bildbereich hineingezogen werden. Ziel dieser Techniken in der allgemeinen Bildbearbeitung ist es zwar, ein überarbeitetes Bild zu erzeugen, an dem der Betrachter nicht erkennen kann, dass Veränderungen vorgenommen wurden, da es sich hierbei jedoch grundsätzlich um künstliche Bildinformation handelt, ist diese Bildinformation grundsätzlich nicht verlässlich. Entsprechend sollten die eingefügten Bildinformationen in der Darstellung für den Benutzer als ungesichert oder unzuverlässig markiert werden.

In einigen Ausführungsformen kann die Bildinformation aus mindestens einem Bild extrahiert werden, das aus einer anderen Perspektive aufgenommen wurde. In einigen Ausführungsformen kann das mindestens eine Bild, das aus einer anderen Perspektive aufgenommen wurde, von einer zweiten Bildverarbeitungsvorrichtung aufgenommen werden. In einigen Ausführungsformen kann, das aus einer anderen Perspektive aufgenommene Bild nach einer Bewegung der medizinischen Bildgebungsvorrichtung aufgenommen werden, wobei die Bewegung bevorzugt durch ein motorisiertes System, wie beispielsweise durch einen Roboterarm durchgeführt werden kann, um so Informationen hinsichtlich der Bewegung zu erhalten und in etwaigen nachfolgenden Bildanalyse zu berücksichtigen. In Ausführungsformen der vorliegenden Erfindung kann die Bewegung senkrecht zur Hauptausrichtung der Informationen erfolgen.

In einigen, bevorzugten Ausführungsformen kann die medizinische Bildgebungsvorrichtung zwei nebeneinander angeordnete Objektive umfassen, mit denen jeweils ein rechtes und ein linkes Bild aufgenommen werden kann, wobei das mindestens eine digitale Bild ein dreidimensionales Bild sein kann. Hier können insbesondere Kamarasysteme mit achsparalleler Ausrichtung also auch solche mit konvergenter Ausrichtung verwendet werden. Aufgrund der unterschiedlichen Perspektiven der beiden Kameras werden Informationen, wie beispielsweise Gegenstände im in der Nähe der Kameras an unterschiedlichen Positionen der jeweiligen Bildebenen abgebildet. Entsprechend werden in dem linken und rechten Bild unterschiedlichen Bereiche des Betrachtungsbereiches im Hintergrund des Bildes von der Information im Vordergrund verdeckt. So kann Bildinformation als zweidimensionale Information aus dem linken Bild und/oder dem rechten Bild extrahiert werden. Bei der Verwendung von Kamerasystem mit konvergenter Ausrichtung ist es vorteilhaft die Bilder zu kalibrieren, um die Zuordnung von Objekten in den jeweiligen Bildern zu erleichtern. Die Kalibrierung erfolgt bevorzugterweise nach den Prinzipien der Epipolargeometrie. Sofern auch Bildinformationen verdeckter Bereiche nicht aus dem linken Bild und/oder dem rechten Bild extrahiert werden können, können in bevorzugten Ausführungsformen die entsprechende Bildinformationen gemäß anderer, wie beispielsweise der vorstehend beschriebenen Verfahren extrahiert und/oder erzeugt werden.
In Ausführungsformen der vorliegenden Erfindung kann der überlagerte Bildbereich als transparent, teilweise transparent und/oder als Umriss dargestellt werden. In einigen Ausführungsformen kann die Art der Darstellung des überlagerten Bildbereichs auch von der Zuverlässigkeit der verwendeten Bildinformationen abhängen. In einigen Ausführungsformen kann die Zuverlässigkeit der verwendeten Bildinformationen durch eine Plausibilitätsprüfung auf der Grundlage vordefinierter Metriken bestimmt werden. Entsprechend kann die Zuverlässigkeit von Bildinformationen, die aus Stereobildpaaren extrahiert wurde als sicher kategorisiert werden, da diese Bildinformationen zeitgleich mit dem Bild erfasst wurden und somit die reale Situation in dem Moment der Bildaufnahme darstellen. Bildinformationen, die aus zeitlich früher aufgenommenen Bilder extrahiert wurden, können je nach Alter der Bilder und/oder auch je nach Bewegung des Betrachtungsbereichs oder der Kamera als zunehmen unsicher kategorisiert werden. Künstliche generierte Bildinformationen können als unsicher kategorisiert werden. In Ausführungsformen der vorliegenden Erfindung kann die Zuverlässigkeit der Überlagerung durch den Vergleich der überlagerten Bildinformationen mit dem mindestens ein Bild bestimmt werden.

In Ausführungsformen der Erfindung kann die Darstellung der Überlagerung die Zuverlässigkeit der Bildinformation widerspiegeln. Entsprechend kann eingefügte Bildinformation in der Darstellung für den Benutzer zum Beispiel durch Entsättigung, oder auch Ausgrauen, als ungesichert oder unzuverlässig markiert werden. Somit kann der Benutzer erkennen, dass ja nach Darstellung die eingefügt Bildinformation beispielsweise nur der geometrisch räumlichen Orientierung und zur Verringerung der Ablenkung durch z.B. Schäfte dienen, nicht aber für irgendwelche informativen, diagnostischen oder gar therapeutische Zwecken verwendet werden können.

In Aspekten betrifft die Erfindung ein System, das mindestens eine medizinischen Bildgebungsvorrichtung, mindestens eine Bildverarbeitungsvorrichtung und mindestens eine Anzeigevorrichtung enthält. In einigen Ausführungsformen kann das System so konfiguriert sein, dass es ein vorstehend beschriebenes ausführen kann. In einigen Ausführungsformen führt die Bildverarbeitungsvorrichtung einen Diskriminator aus, um die Zuverlässigkeit von Bildinformationen zu bestimmen, die durch auf künstlicher Intelligenz basierende generative Bildverarbeitungsmethoden erzeugt wurden.

Ein Aspekt der Erfindung betrifft ein Computerprogramm mit Anweisungen, die, wenn das Programm von einem Computer ausgeführt werden kann, den Computer veranlassen, eines der vorstehend beschriebenen Verfahren auszuführen.

Ein Aspekt der Erfindung betrifft ein computerlesbares Speichermedium, das Anweisungen enthält, die, wenn sie von einem Computer ausgeführt werden, den Computer veranlassen, die Verfahren der Punkte 1 bis 14 auszuführen.

### Kurzbeschreibung der Abbildungen

Weitere Aspekte der Erfindung ergeben sich aus der nachfolgenden Beschreibung bevorzugter Ausführungsbeispiele und der beigefügten Abbildungen. Hierbei zeigen:
Abbildungen 1a, 1b die Aufnahme von Stereobildpaaren mit achsparallel ausgerichteten Kameras;
Abbildungen 2a, 2b ein achsparalleles Kamerasystem sowie die Abbildung eines Punktes in den entsprechenden Bildebenen;
Abbildungen 3a, 3b, 3c ein konvergentes Kamerasystem, die Abbildung eines Punktes in den entsprechenden Bildebenen sowie ein kalibriertes Bildpaar;
Abbildung 4 den Ablauf des erfindungsgemäßen Verfahrens;
Abbildung 5 den Ablauf der alternativen Verfahren zur Bestimmung von Bildinformation;
Abbildung 6 den Ablauf des erfindungsgemäßen Verfahrens zur Überlagerung basierend auf Stereobildpaaren;
Abbildungen 7a, 7b ein Stereobildpaar;
Abbildungen 7c, 7d das Stereobildpaar nach der Kalibrierung;
Abbildung 8a, 8b Tiefenkarten der kalibrierten Stereobilder;
Abbildungen 8c, 8d Segmentierung der Tiefenkarten;
Abbildungen 9a, 9b die Überlagerung der korrespondierenden Bildinformation.

### Detaillierte Beschreibung

Es ist die Grundlage der Erfindung, aus unterschiedlichen Orts- und/oder Zeit-Perspektiven (noch) verfügbare, aber im aktuellen Bild durch Informationen, wie zum Beispiel Instrumente oder auch Reflexionen, verdecktes Hintergrundgewebe, gegebenenfalls perspektivisch korrigiert, zu ergänzen, sowie die der jeweiligen Methode entsprechende Wahrscheinlichkeit, dass diese Ergänzung der Realität entspricht angemessen zu berechnen und diese Wahrscheinlichkeit entsprechend darzustellen.

Für eine stereographische Darstellung von Betrachtungsbereichen sind zwei Bilder desselben Betrachtungsbereichs, die, von zwei verschiedenen, um eine bestimmte Strecke - die Basis - voneinander entfernten Positionen aufgenommen wurden erforderlich. Stereoaufnahmen können auf unterschiedliche Weise generiert werden. Verwendet man nur eine Kamera, werden aufeinanderfolgende Bilder einer Sequenz als Stereobilder herangezogen; durch Bewegung der Kamera wird gewissermaßen ein Stereokamerasystem simuliert. Bei Festbasisstereokameras sind zwei Kameras mit festem Abstand zueinander angeordnet, die Basislänge vorgegeben und Kameras produzieren Stereobildpaare in achsenparalleler Anordnung. Dabei stehen die Aufnahmerichtungen normal zur Basislinie und sind zueinander parallel, wodurch die Stereoanalyse vereinfacht wird.

Entsprechend setzen verschiedene Stereoalgorithmen diese geometrische Anordnung der Kameras voraus. Alternativ dazu werden auch Kamerasysteme mit konvergenter Anordnung verwendet. Dies kann beabsichtigt oder auch system- oder produktionsbedingt sein; diese Konvergenz kann allerdings in einem Nachbearbeitungsschritt durch eine virtuelle Drehung der Bilder ausgeglichen werden. Diesen Prozess bezeichnet man als (epipolare) Begradigung oder Rektifikation oder auch als Kalibration.

Es ist die Zielsetzung von Stereokamerasystemen, virtuell oder real, Bilder in achsenparalleler Anordnung zu liefern, bei denen die Aufnahmerichtungen normal zur Basislinie und zueinander parallel sind, wodurch eine nachfolgende Stereoanalyse vereinfacht wird.

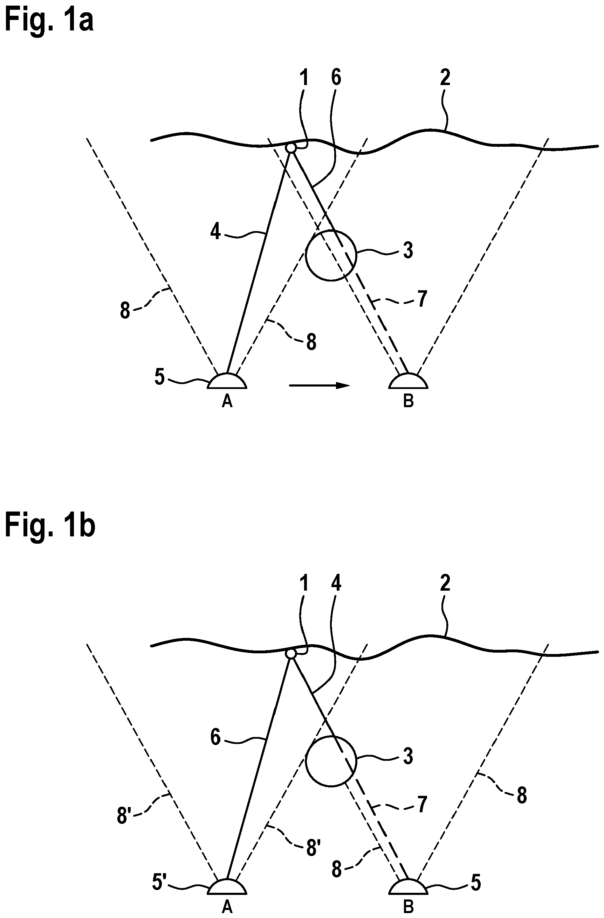

Abbildung 1a zeigt ein Szenario, in dem mittels einer einzigen Kamera 5 in Position A ein erstes Bild von einem Betrachtungsbereich aufgenommen wird. Der Betrachtungsbereich umfasst einen Hintergrund 2 und ein Objekt 1. Die Linien 8 beschreiben den Blinkwinkel und damit das Blickfeld der Kamera und die Linie 4 stellt den Strahl zur Abbildung des Objekts 1 dar. In Position A der Kamera ist bei dem gegebenen Blickwinkel der Kamera 5 der Gegenstand 3 nicht im Blickfeld der Kamera 5. Anschließend wir die Kamera in Position B bewegt und ein zweites Bild von dem Betrachtungsbereich aufgenommen. In Position B gerät der Gegenstand 3 in das Blickfeld der Kamera und verdeckt somit in der Abbildung Teile des eigentlichen Betrachtungsbereichs, nämlich unter anderem das Objekt 1, wie mit der Darstellung des Strahls 6, 7 angedeutet, sowie Teile des Hintergrunds 2.

Abbildung 1b zeigt schematisch ein entsprechendes Szenario zur Aufnahme von zwei Stereobilder von einem Objekt 1 vor einem Hintergrund 2 durch zwei Kameras 5, 5' mit achsparalleler Anordnung, wobei ein Gegenstand 3 im Vordergrund angeordnet ist. Hierbei beschreiben die Linien 8 und 8' jeweils die Blinkwinkel der Kameras 5, 5' und die Linien 6 und 7 stellen die Strahlen zur Abbildung des Objekts 1 auf den jeweiligen Kameras 5, 5' dar. Der Strahl 6 kann ungehindert die Kamera 5' erreichen, wobei der Strahl 4 durch den Gegenstand geblockt wird, die hypothetische Weiterführung 7 bis zur Kamera 5 führt also nicht zu einer Abbildung des Objekts 1 auf der Kamera 5. In der Überlagerung der beiden Stereobilder für eine dreidimensionale Darstellung des Betrachtungsbereichs umfassend das Objekt 1 und den Hintergrund 2, wird also ein Teil des Betrachtungsbereichs von dem Gegenstand 3 verdeckt.

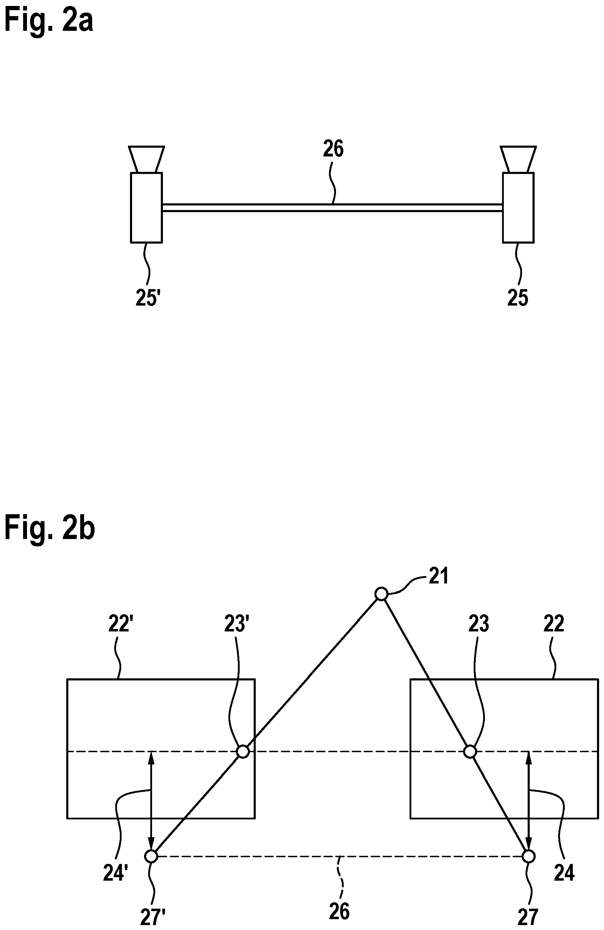

Abbildungen 2a und 2b zeigen eine schematische Darstellung eines achsparallelen Stereosystems zur Aufzeichnung von Stereobildpaaren. Das System zeichnet sich durch zwei Kameras 25, 25' aus, die horizontal um die Basislänge 26 verschoben angeordnet sind; die Bildebenen 22, 22' der Kameras 25, 25' - und somit deren Koordinatensysteme - sind nicht gegeneinander verdreht. In Abbildung 2b ist die Frontsicht auf das achsparallele System von Abbildung 2a dargestellt. Die Bildebenen 22, 22' sind parallel und die beiden optischen Zentren 27, 27' horizontal um die Basislänge 26 verschoben. Die Brennweite 24, 24' legt den Abstand der beiden Brennpunkte zu ihren Bildebenen 22, 22' fest und wird in der Darstellung für beide Kameras als identisch vorausgesetzt. Ein Objekt 21 wird somit über die beiden optischen Zentren in die Bildpunkte 23, 23' in den jeweiligen Bildebenen 22, 22'projiziert. Da bei einem achsparallelen Stereosystem die Bildzeilen in den jeweiligen digitalen Bildern identisch sind, führt die unterschiedliche Perspektive der Kameras hinsichtlich des Objekts 21 zu einem rein horizontalen Versatz der Bildpunkte 23, 23' in deren Abbildung. Dieser Unterschied wird als Disparität bezeichnet und stellt die relative Verschiebung der Abbildung des Objekts 21 dar. Wenn sich Gegenstände zwischen den Kameras und den abzubildenden Strukturen befinden, kann die optische Abbildung, wie im Zusammenhang mit Abbildung 1 beschrieben, gestört beziehungsweise überlagert werden.

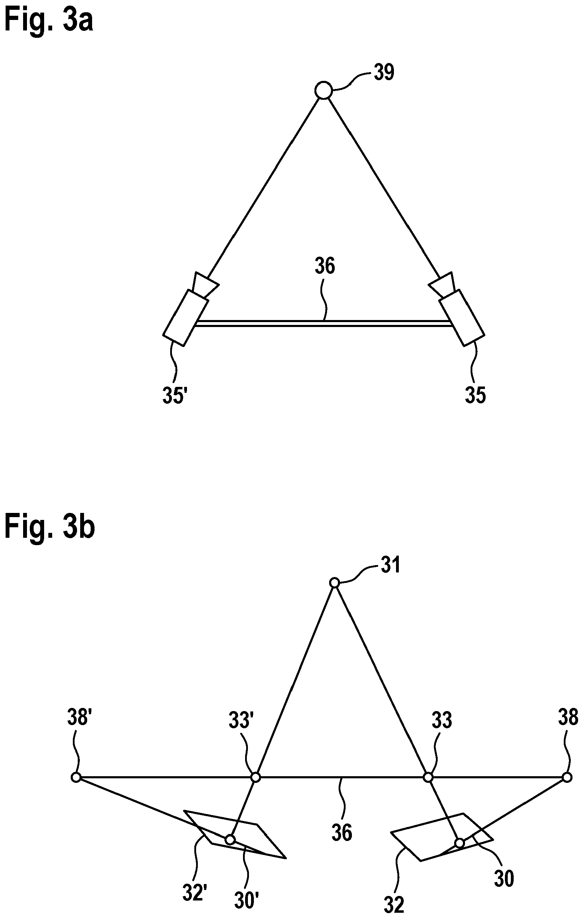

Abbildungen 3a bis 3c zeigen eine schematische Darstellung eines Stereosystems mit konvergenter Anordnung der um die Basislänge 36 verschobenen Kameras 35, 35'; die optischen Achsen der Kameras 35, 35'sind auf einen Konvergenzpunkt 39 ausgerichtet. Entsprechend sind die beiden Kameras 35, 35' - anders als im Fall des achsparallelen Systems von Abbildung 2a und 2b - nicht nur horizontal verschoben, sondern auch noch zueinander gedreht. Solche Verdrehungen bezüglich der Ausrichtung der Kamera können gewollt, system- oder auch produktionsbedingte Abweichungen von einer achsparallelen Ausrichtung sein.

Mittels des mathematischen Modells der Epipolargeometrie, das die geometrischen Beziehungen zwischen verschiedenen Kamerabildern desselben Objekts beschreibt, lässt sich die Abhängigkeit zwischen korrespondierenden Bildpunkten - also den Punkten, die ein einzelner Objektpunkt in den beiden Kamerabildern erzeugt - beschreiben.

Um Zuordnungen von Informationen zwischen den beiden Bildebenen, beziehungsweise zwischen den mit den beiden Kameras 35, 35'aufgenommenen Bildern, zu ermöglichen, sollten die jeweiligen Bilder rektifiziert werden. Ziel der Rektifikation eines Stereobildpaares ist die Simulation eines perfekten Stereokamerasystems, bei dem die optischen Achsen parallel verlaufen und die Bildebenen koplanar sind.

In der Epipolargeometrie wird die sogenannte Epipolarebene durch die Projektionszentren der beiden Kameras 33, 33' sowie einem Bildpunkt 31 definiert. Die Epipolarlinien 30, 30'ergeben sich aus der Schnittfläche der Epipolarebene mit den Bildebenen 32, 32' der Kameras 33, 33'. Alle Epipolarlinien einer Bildebene 32, 32'schneiden sich in den Epipolen 38, 38'. Ändert sich der Bildpunkt 31, entsteht eine andere Epipolarebene und damit ebenfalls andere Epipolarlinien. Abbildung 3b zeigt schematisch den Verlauf der Epipolarlinien 30, 30'quer durch die Bildflächen 32, 32' der Kameras 33, 33'in konvergenter Anordnung.

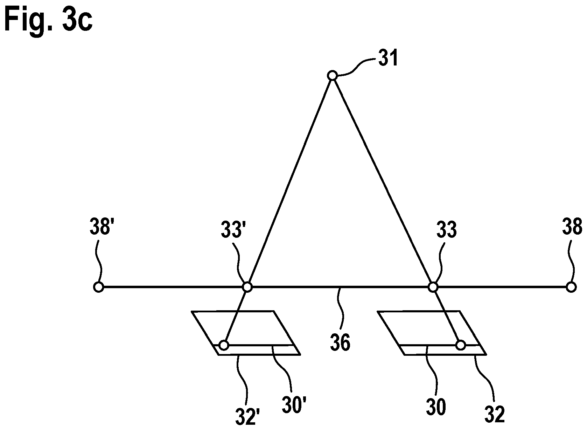

Durch die Rektifikation wird nun die konvergente Kameraanordnung mathematisch in ein achsenparalleles Stereosystem umgewandelt. Wie in Abbildung 3c schematisch dargestellt, werden durch die Rektifikation die Epipolarlinien 30, 30'parallel zueinander ausgerichtet, wodurch die Epipole 38, 38' ins Unendliche wandern. Die Abbildung korrespondierender Bildpunkte in den Bildern liegt nun immer auf korrespondierenden Zeilen in den entsprechenden Bildern. Die Korrespondenzsuche reduziert sich nach der Rektifikation auf die Suche zwischen einer Zeile im linken Bild und der entsprechenden Zeile im rechten Bild.

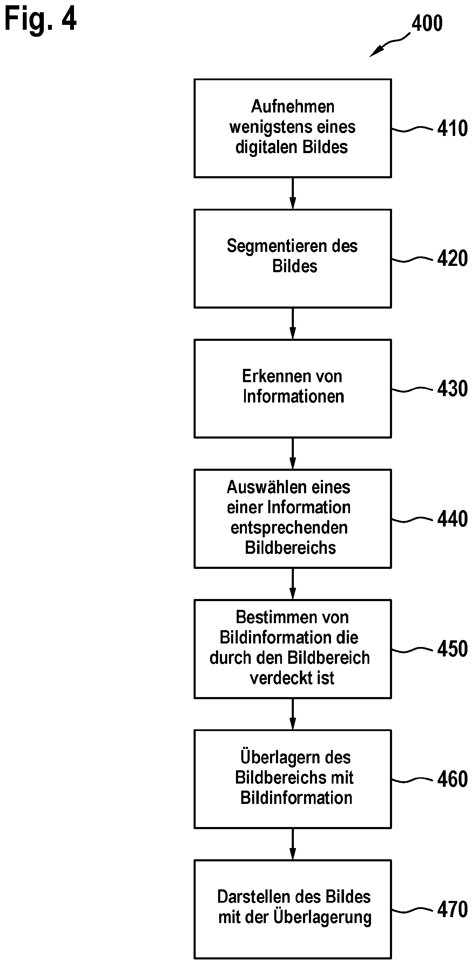

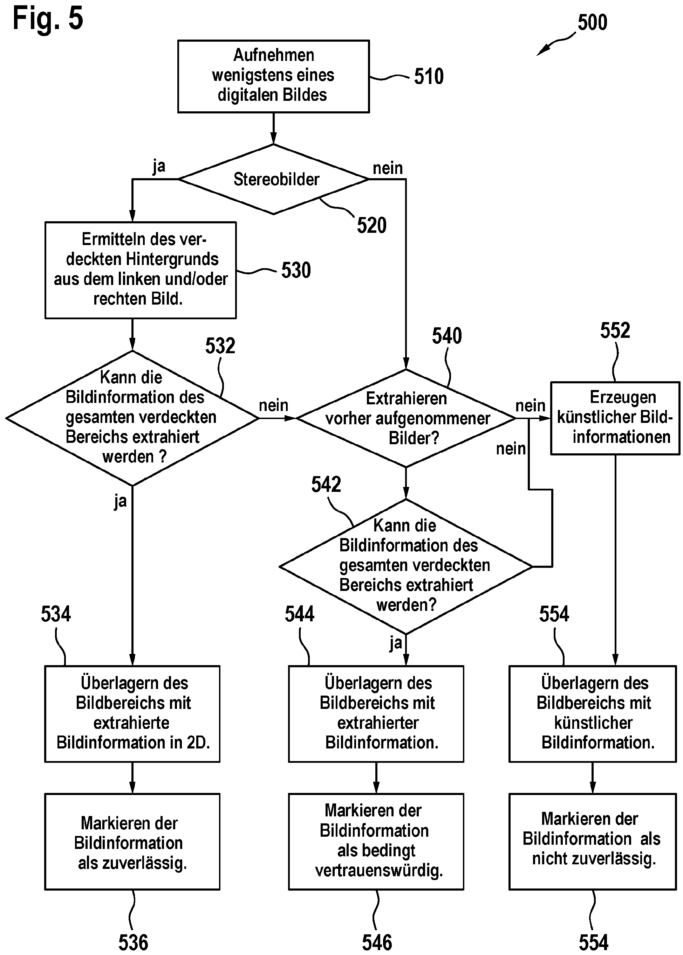

Abbildung 4 beschreibt die Grundzüge des erfindungsgemäßen Verfahrens 400. In einem ersten Schritt 410 des Verfahrens wird mittels einer medizinischen Bildgebungsvorrichtung, wie beispielsweise einer Endoskop-Kamera, ein digitales Bild aufgenommen. In Abhängigkeit davon, welche Art Bildmaterial aufgenommen wird, wie zum Beispiel Stereobildpaare, Einzelbilder, Videoframes, etc., können sich einzelne Verfahrensschritte, wie in Zusammenhang mit Abbildung 5 im Detail beschrieben, unterscheiden. Die grundsätzlichen mit Abbildung 4 beschriebenen Verfahrensschritte finden jedoch weitgehend universelle Anwendung.

In dem darauffolgenden Schritt 420 wird das Bild segmentiert, es werden also inhaltlich zusammenhängende Regionen durch Zusammenfassung benachbarter Pixel entsprechend einem bestimmten Homogenitätskriteriums erzeugt. Grundsätzlich kann die Segmentierung im Rahmen der vorliegenden Erfindung auf verschiedene Weise, insbesondere mittels pixel-, kanten- und regionenorientierter Verfahren erfolgen. Zusätzlich kann zwischen modellbasierten Verfahren, bei denen von einer bestimmten Form der Objekte ausgegangen wird, und texturbasierten Verfahren, bei denen auch eine innere homogene Struktur der Objekte berücksichtigt wird, unterschieden werden. Grundsätzlich können alle der vorgenannten Verfahren, auch in Kombination, im Rahmen der vorliegenden Erfindung Anwendung finden. Insbesondere kann die Segmentierung auch mit Hilfe künstlicher Intelligenzalgorithmen erfolgen. Hierbei ist es insbesondere vorteilhaft, wenn der Algorithmus mit Gegenständen trainiert wurde, die in den Bildern der nachfolgenden Anwendung der Verfahren enthalten sein können. So ist es bei der Verwendung von Endoskopen bei medizinischen Eingriffen vorteilhaft, wenn der Algorithmus die verschiedenen Instrumente, die in einem medizinischen Eingriff verwendet werden, kennt, um so die Qualität der Segmentierung zu verbessern.

Nachfolgend können in Schritt 430 des Verfahrens bestimmte Informationen, die segmentierten Bereichen zugeordnet werden können, erkannt und gegebenenfalls identifiziert werden. Diese Erkennung kann gegebenenfalls manuell, halb-automatisch oder automatisch erfolgen und kann gegebenenfalls auch unter Verwendung künstlicher Intelligenzalgorithmen erfolgen. Gegebenenfalls können die Segmentierung und die Erkennung gleichzeitig erfolgen.

Insbesondere können sogenannte Mehrklassen oder multiclass Segmentierungsalgorithmen verwendet werden, bei denen die segmentierten Bildbereiche bestimmen Klassen zugeordnete werden. Im Beispiel des endoskopischen, medizinischen Eingriffs können beispielsweise die Klassen Gewebe, Arbeitselement (also der Werkzeugbereich eines endoskopischen Instruments) und Instrumentenschaft verwendet werden. Somit können segmentierte Bildbereiche gleich zugeordnet und erkannt werden und basierend auf dieser Zuordnung können durch das erfindungsgemäße Verfahren bestimmte Bildbereiche, wie zum Beispiel der Instrumentenschaft, überlagert und somit transparent dargestellt werden.

Nachfolgend kann in Schritt 440 des Verfahrens ein Bildbereich, der einer bestimmten, erkannten Information entspricht, als der Bildbereich ausgewählt werden, der in den nachfolgenden Verfahrensschritten mit Bildinformation überlagert werden soll. Diese Auswahl kann manuell, also durch den Benutzer des Verfahrens oder auch automatisch, zum Beispiel in Abhängigkeit der Erkennung erfolgen. So kann, wieder am Beispiel der Verwendung von Endoskopen bei medizinischen Eingriffen, ein bestimmtes Instrument oder ein Teil eines Instruments, wie ein Instrumentenschaft, automatisch für eine nachfolgende Überlagerung ausgewählt werden.

Nachfolgend wird in Schritt 450 die Bildinformation des Betrachtungsbereichs ermittelt, die von dem Gegenstand im Vordergrund, der in den vorstehenden Schritten erkannt wurde, verdeckt wird. Für die Ermittlung der Bildinformation können erfindungsgemäß verschiede Verfahren angewandt werden. So kann die Bildinformation erfindungsgemäß aus einem Stereobildpaar extrahiert werden, sofern der verdeckende Gegenstand so angeordnet ist, dass entsprechende Abschnitte des Betrachtungsbereichs jeweils nur von einem der beiden Bilder verdeckt sind. Dieses Verfahren beruht auf der unterschiedlichen Orts-Perspektive der beiden Kameras, da diese, wie vorstehend beschrieben, zumindest horizontal beabstandet, gegebenenfalls zusätzlich konvergent angeordnet sind und sich somit im Blickfeld unterscheiden. Da die so extrahierte Bildinformation zeitgleich aufgenommen wurde, entspricht der Inhalt exakt dem, was durch den Gegenstand im Vordergrund verdeckt wurde; es besteht somit keinerlei Unsicherheit hinsichtlich der Verlässlichkeit des Inhalts. Details dieses Verfahrens werden im Zusammenhang mit Abbildung 5 beschrieben.

Alternativ oder in Ergänzung dazu, beispielsweise wenn nicht die gesamte Bildinformation des verdeckten Bereichs aus dem Stereobildpaar extrahiert werden kann, kann Bildinformation aus einer unterschiedlichen Zeit-Perspektive, also aus vorher aufgenommenen Bildern, extrahiert werden. In vielen Fällen erfolgt die Visualisierung des Betrachtungsbereichs in Form von Videoframes, also zeitlich aufeinanderfolgenden Bildern des Betrachtungsbereichs. Je nach Situation, kann es gegebenenfalls ältere Bilder geben, in denen der in dem aktuellen Bild verdeckte Bereich des Betrachtungsbereichs nicht verdeckt ist. Dies kann zum einen auf eine Bewegung der Kamera zurückzuführen sein. Entsprechend kann beispielsweise der im aktuellen Bild in der Bildmitte befindliche, und dort durch einen Gegenstand verdeckte Bereich, in älteren Bildern, vor Bewegung der Kamera, am Bildrand gewesen sein. Zum anderen oder in Ergänzung dazu, kann der verdeckende Gegenstand in älteren Bildern noch nicht im Blickfeld der Kamera gewesen sein. Dies kann am Beispiel des endoskopischen medizinischen Eingriffs zum Beispiel durch das Einbringen eines Instruments geschehen. Aufgrund des zeitlichen Abstands zwischen der Aufnahme des Bildes, aus dem die Bildinformation extrahiert wird, und dem aktuellen Bild, in das die Bildinformation eingesetzt werden soll, besteht eine gewisse Unsicherheit hinsichtlich der Verlässlichkeit des Inhalts, die abhängig vom Alter des Bildes ist, aus dem die Bildinformation extrahiert wird. Details dieses Verfahrens werden im Zusammenhang mit Abbildung 5 beschrieben.

Alternativ, oder in Ergänzung dazu, beispielsweise wenn nicht die gesamte Bildinformation des verdeckten Bereichs aus dem Stereobildpaar, oder aus älteren Bildern extrahiert werden kann, kann Bildinformation künstlich erzeugt werden. Hierfür können, wie im Detail in Zusammenhang mit Abbildung 5 beschrieben, künstliche Intelligenzalgorithmen, wie beispielsweise InPaint-Algorithmen verwendet werden. Da es sich bei der künstlich erzeugten Bildinformation um rein synthetische Daten handelt, die zwar optisch in das Bild passen, jedoch keine reale Bildinformation wiedergeben, ist diese Bildinformation grundsätzlich unsicher hinsichtlich der Verlässlichkeit des Inhalts. Details dieses Verfahrens werden im Zusammenhang mit Abbildung 5 beschrieben.

Nachdem die Bildinformation, wie vorstehend beschrieben extrahiert, und/oder generiert wurde, wird im nächsten Schritt 460 der nach der Segmentierung ausgewählte Bildbereich mit dieser Bildinformation überlagert, wobei, sofern nötig, perspektivische Anpassungen der Bildinformation vorgenommen werden können. Durch die Überlagerung wird der verdeckende Gegenstand quasi transparent dargestellt. Hierbei kann durch die Darstellung der Überlagerung eine Indikation für die Verlässlichkeit der für die Überlagerung verwendeten Bildinformation gegeben werden. So kann die eingefügte Bildinformation in der Darstellung für den Benutzer als ungesichert oder unzuverlässig, zum Beispiel durch Entsättigung, oder auch Ausgrauen, markiert werden. Somit kann der Benutzer erkennen, dass die eingefügten Bildinformationen nur der geometrisch räumlichen Orientierung und zur Verringerung der Ablenkung durch z.B. Schäfte dienen, nicht aber für irgendwelche informativen, diagnostischen oder gar therapeutische Zwecken verwendet werden können.

Abbildung 5 beschreibt das erfindungsgemäße Verfahren 500 unter Einbeziehung der verschiedenen Möglichkeiten, um in einem aktuellen Bild verdeckte Bildbereiche durch entsprechende Bildinformation zu ergänzen. Grundsätzlich können die einzelnen in Abbildung 5 beschriebenen Möglichkeiten, insbesondere die Extraktion von Bildinformation aus Stereobildpaaren, beziehungsweise aus Bildern, die mit einer weiteren medizinischen Bildgebungsvorrichtung aus einer anderen Perspektive aufgenommen wurden, die Extraktion von Bildinformation aus vorherigen Bildern oder die Generation von künstlicher Bildinformation unabhängig voneinander oder auch in Kombination miteinander angewandt werden. In Ausführungsformen der vorliegenden Erfindung können die beiden medizinischen Bildgebungsvorrichtungen zusammen ein eine Stereokamera, vorzugsweise eine Stereoenddoskopkamera darstellen.

Zu Beginn des Verfahrens 500 wird in einem ersten Schritt 520 wenigstens ein digitales Bild durch eine entsprechende medizinischen Bildgebungsvorrichtung, wie beispielsweise eine Endoskop-Kamera, aufgenommen. In Abhängigkeit von der medizinischen Bildgebungsvorrichtung kann es sich hierbei um ein Einzelbild handeln, oder im Fall einer Stereokamera, wie beispielsweise ein Stereo-Endoskop, kann es sich um ein Stereobildpaar, also um gleichzeitig aus unterschiedlichen Perspektiven sich teilweise überschneidende Bilder, handeln. Im nächsten Schritt 520 des Verfahrens wird entsprechend unterschieden, ob es sich um ein Einzelbild oder um ein Stereobildpaar handelt.

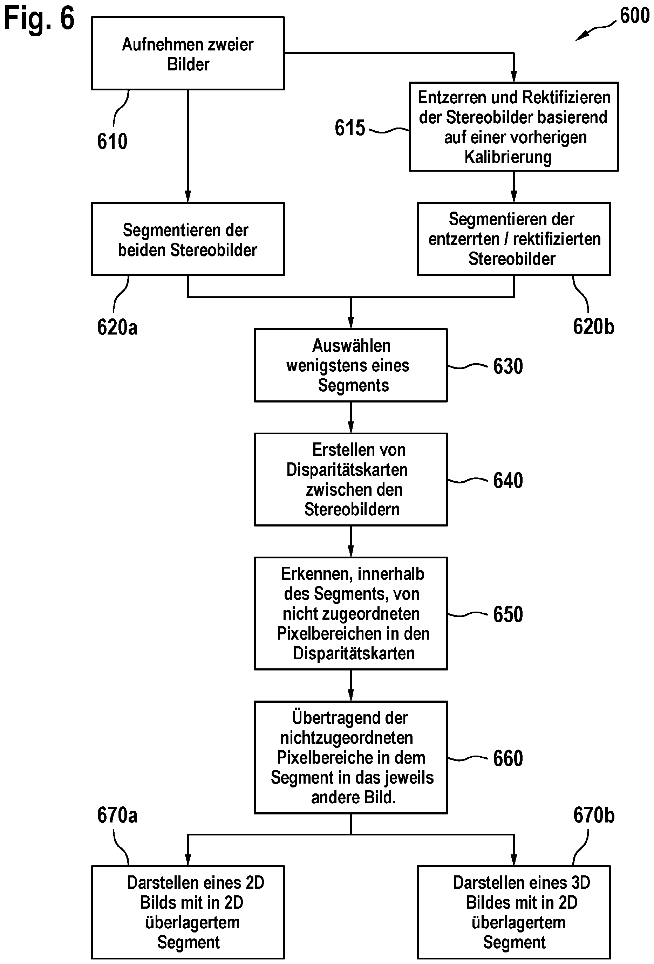

Im Fall eines Einzelbildes wird das Verfahren mit Schritt 540 fortgeführt, welcher im Detail später beschrieben wird. Im Fall eines Stereobildpaars wird das Verfahren mit Schritt 530 fortgeführt, in dem die Bilder segmentiert werden und der verdeckte Hintergrund in den beiden Bildern ermittelt wird. Details dieses Verfahrensschritts werden in Abbildung 6 beschrieben. Basierend auf dem Ergebnis dieses Verfahrensschritts 530, wird nachfolgend in Schritt 532 überprüft, ob Bildinformationen für den gesamten verdeckten Bereich aus dem Stereobildpaar extrahiert werden kann. Wenn der verdeckende Gegenstand in der Bildaufnahme zu groß, zu nah an der Kamera und/oder zu nah am Hintergrund ist, ist die Disparität zu klein und die verdeckte Bildinformation kann nicht vollständig aus dem Stereobildpaar extrahiert werden. In diesem Fall kann entweder der verdeckende Gegenstand nicht vollständig überlagert werden, oder die Bildinformation kann auf anderem Wege, wie beispielsweise aus vergangenen Frames extrahiert, oder mittels künstlicher Intelligenz Algorithmen generiert werden. Entsprechende Verfahrensschritte 540, 552 werden später im Detail beschrieben.

Sofern sich aus der Überprüfung in Schritt 532 ergibt, dass die Bildinformation des gesamten verdeckten Bereichs aus dem Stereobildpaar extrahiert werden kann, wird die extrahierte Bildinformation in Schritt 534 in das entsprechende Segment eingesetzt, der verdeckende Gegenstand also überlagert, und somit transparent dargestellt. Da die verwendete Bildinformation nur jeweils aus einem der beiden Stereobilder entnommen wird (beziehungsweise sich aus Informationen des Linken und des rechten Stereobildes zusammensetzt), ist die Darstellung dieses Bereiches zweidimensional. Der durch das Verfahren "unsichtbar" gemachte Gegenstand kann in der Darstellung auf verschiedene Weisen dargestellt werden. Entsprechend kann der Gegenstand komplett transparent, also vollständig ausgeblendet werden. Alternativ dazu kann der Umriss des Gegenstandes dargestellt werden oder der Gegenstand kann, beispielsweise durch alpha-blending, teiltransparent dargestellt werden.

Sofern nötig kann die Bildinformation perspektivisch an das Segment, in das die Information eingesetzt werden soll, angepasst werden.

Da es sich bei der Bildinformation um reale, zweitgleich aufgenommene Information handelt, besteht keine Unsicherheit hinsichtlich des Inhalts. Entsprechend kann in einem optionalen Schritt 536 die Bildinformation in der Darstellung auf einem Ausgabegerät, wie einem Monitor oder einer Videobrille als sicher markiert werden. Für eine solche Markierung der Zuverlässigkeit können verschiedene Methoden verwendet werden. Beispielsweise kann die eingesetzte Bildinformation mit abnehmender Zuverlässigkeit des Inhalts zunehmend entsättigt oder auch teilweise ausgegraut werden.

Wenn, wie vorstehend beschrieben, entweder keine Stereobildpaare vorliegen oder nicht die gesamte Bildinformation des verdeckten Bereichs extrahiert werden kann, kann das in den Schritten 540 bis 546 beschriebene Verfahren ausgeführt werden. Hier wird in Schritt 540 überprüft, ob wenigstens ein vorher aufgenommenes Bild verfügbar ist. Hierfür finden insbesondere Bilder Verwendung, die vor der Bewegung der medizinischen Bildgebungsvorrichtung aus einer anderen Perspektive oder bevor der Verdeckende Gegenstand in das Blickfeld gekommen ist, aufgenommen wurden. So kann beispielsweise aus einem vergangenen Frame einer Videoaufnahme, die mittels einer Endoskop-Kamera aufgenommen wurden, nach Änderung der Perspektive der Kamera und/oder der Position des Gegenstands, die Bildinformation, der im aktuellen Bild durch den Gegenstand verdeckten Bereiche gegebenenfalls noch extrahiert werden. Hierbei kann das System insbesondere so konfiguriert sein, dass entsprechende ältere Bilder gespeichert bzw. für eine erfindungsgemäße Verwendung vorgehalten werden.

Maßgeblich für die Überlagerung von Bildbereichen in einem Bild mit Bildinformationen, die aus einem älteren Bild extrahiert wurden ist die Ausrichtung, das sogenannte Alignement, der Bildinformationen beziehungsweise der Bilder. Hierbei kann die Bewegung beispielsweise über sogenannte optische Fluss-Algorithmen bestimmt und nachvollzogen werden. Basierend darauf können Bewegungskorrekturen durchgeführt werden.

Dazu oder daneben können auch verschiedene Metaparameter, wie u.a. Schärfe, feiner Detailgehalt, lokaler Kontrast für ein pixelgenaues Matching genutzt werden. Darüber hinaus kann der Aufnahmezeitpunkt - und damit das Alter des Bildes - als Indikator für die Wahrscheinlichkeit genutzt werden, dass die ergänzte Fläche noch akzeptabel aktuell ist. Insbesondere kann der Aufnahmezeitpunkt auch in Kombination mit Bewegungsinformationen als Indikator für die Wahrscheinlichkeit genutzt werden, dass die ergänzte Fläche noch akzeptabel aktuell ist. Die für die Überblendung eingesetzte Bildinformation kann entsprechend der bestimmten Zuverlässigkeit der Information dargestellt werden; so kann die Information beispielsweise entsättigt oder ausgegraut dargestellt werden.

In Ausführungsformen der Erfindung können als Burst-Imaging bekannte Techniken genutzt werden, bei denen bewegungskorrigierte und bereichsweise realisierte "Langzeitbelichtung" verwendet werden, um aus einem oder typischerweise mehreren älteren Frames auch in den dunklen Bildbereichen Rausch-verringerte hellere Texturen mit erhöhter Dynamik und verringerter Bewegungsunschärfe zu erzeugen. Gleichermaßen können durch Überlagerungen von Informationen, wie verdeckende Gegenstände, diese ausgeblendet werden. Je nach Anwendung oder Konfiguration können somit verdeckende Gegenstände oder Information (wie Reflexionen, überblendete Bilder oder Bildbereiche, etc.) überblendet und darüber die Bildqualität verbessert werden.

Burst-Imaging nutzt diese bewegungskorrigierte und bereichsweise realisierte "Langzeitbelichtung", um aus einem oder typischerweise mehreren älteren Frames auch in den dunklen Bildbereichen Rausch-verringerte hellere Texturen mit erhöhter Dynamik und verringerter Bewegungsunschärfe zu erzeugen.

Für jedes Tile des aktuellen und jedes Tile der älteren bewegten Frames werden für eine gute Bildqualität mehrere Parameter erfasst: Schärfe und Detailgehalt in den Bewegungen sollten mindestens pixelgenau getrackt werden. Ebenso reduziert das Alter der Frames die Treffergenauigkeit und stellt den 3. Faktor für die Zuverlässigkeit der Information dar. Entsprechend sollte der positive Faktor Schärfe mit zunehmendem Alter von Frames geringer gewertet werden. Umgekehrt ist geringer Detailgehalt positiv, da man weniger Fehlerrisiken mit dem jeweiligen Tile hat. Dies zusammen ergibt die darzustellende Zuverlässigkeit der Bildinformation.

Bei der Anwendung des Burst-Imaging-Verfahrens kann die Qualität einzelner Bild-Tiles in der Image-Pyramide für unterschiedliche Auflösungsstufen erfasst und gewichtete Qualitätsmetriken bestimmt werden. Diese Metriken bilden die Grundlage, um bei ungünstig wechselhaften Lichtverhältnissen schärfere Bildergebnisse zu berechnen und Fehler wie Fehl-Alignement auf mindestens Pixelgenauigkeit zu vermeiden.

Erfindungsgemäß können diese Qualitätsmetriken für eine Zuverlässigkeitsdarstellung wie folgt verwendet werden: Der Aufnahmezeitpunkt kann deutlich länger zurück liegen als für das Livebild Burst-Imaging sinnvoll. Bei Livebild sind 1-5 Frames sinnvoll je nach HDR-Scene bis zu 10 Frames, d.h. bei 60Fps: 16,6ms - 83ms bis im Extremfall 166,6ms. Für das erfindungsgemäße Verfahren zum Überlagern von Informationen werden diese aufgrund der Qualitätsmetriken auf die optisch besten Frames ausgedünnt aber z.B. bis 1 oder 5 Sekunden lang vorgehalten. Dieser Maximalzeitraum ergibt sich dynamisch über den Vergleich älterer mit dann neu erfassten Tiles. Diese älteren Tiles werden stark zunehmend als möglicherweise zunehmend "veraltet" dargestellt aber solange verwendet bis neuere für diese Hintergrund-Position, z.B. durch Bewegung, erfasst worden sind. Alternativ in einer weniger bewegten Scene sollten zu alte z.B. > 5 sec alte Tiles zusätzlich zum Entsättigen geblurrt werden, um erkennbarer zu sein und Rand-matching wegen zu großen Änderungen weniger störend darzustellen.

Wenn, wie vorstehend beschrieben, keine oder keine vollständige Bildinformation des verdeckten Bereichs extrahiert werden kann, können in Schritt 552 mittels auf künstliche Intelligenz basierenden Algorithmen, wie beispielsweise InPainting oder OutPainting, optisch passende, künstliche Bildinformationen generiert werden. Dieses Verfahren wird insbesondere dann ausgeführt, wenn für den zu überlagernden Bildbereich, die Verfügbarkeit von Bildinformationen basierend auf Stereobildpaaren und/oder vorherigen Frames bestimmt wurde, und übrigbleibende Bereiche identifiziert wurden, die mit diesen Methoden nicht mehr akzeptabel, das heißt, mit als sicher eingestufter Bildinformation, ersetzt werden konnten.

Hierfür steht je nach Konfiguration auf künstlicher Intelligenz basierendes InPainting zur Verfügung. Der Vorgang basiert auf einem sukzessiven Fortführen der Randinformationen in das Bildinnere hinein, um so die durch die Verdeckung des Gegenstandes entstandene Lücke in der Darstellung zu füllen. Die angewandten Algorithmen verlängern beispielsweise Linien gleicher Grauwerte am Rand iterativ in den zu füllenden Bereich hinein. Anschließend könne Farben in den zu füllenden Bildbereich hineingezogen werden.

Ziel dieser Techniken in der allgemeinen Bildbearbeitung ist es zwar, ein überarbeitetes Bild zu erzeugen, an dem der Betrachter nicht erkennen kann, dass Veränderungen vorgenommen wurden, da es sich hierbei jedoch grundsätzlich um künstliche Bildinformation handelt, ist diese Bildinformation grundsätzlich nicht verlässlich. Entsprechend sollten die eingefügten Bildinformationen in der Darstellung für den Benutzer als ungesichert oder unzuverlässig markiert werden. Die eingefügten Bildinformationen dienen nur der geometrisch räumlichen Orientierung und zur Verringerung der Ablenkung durch z.B. Schäfte, aber nicht irgendwelchen informativen, diagnostischen oder gar therapeutischen Zwecken.

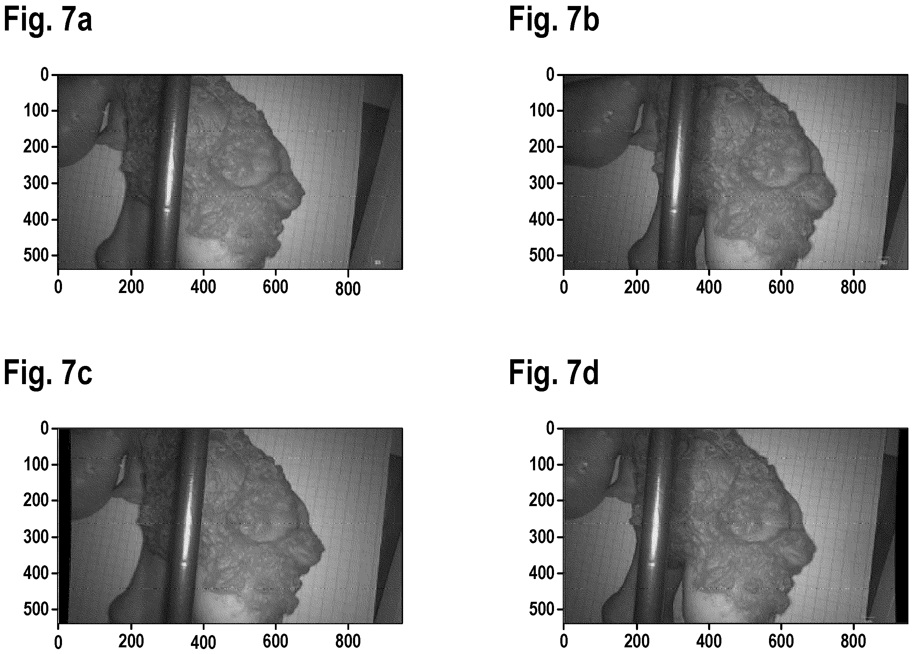

Abbildungen 7 bis 9 zeigen die Anwendung des erfindungsgemäßen Verfahrens zur Überblendung eines Instrumtentenschafts basierend auf stereoendoskopischen Aufnahmen.

Hierbei zeigen Abbildungen 7a, 7b ein linkes und ein rechtes mit einem Stereoendoskop aufgenommenes Bild. Die Bilder zeigen im Vordergrund den Schaft eines Instruments. Im Hintergrund des Bildes befindet sich ein Modell eines Gewebes auf einem karierten Hintergrund. Es ist erkennbar, dass in dem linken und dem rechten Bild unterschiedliche Bereiche des Gewebes durch den Instrumentenschaft verdeckt werden. Die Linien des Hintergrunds sind insbesondere in den Randbereichen der Bilder leicht verzerrt, was eine Zuordnung von Strukturen, beziehungsweise Pixeln in dem jeweils anderen Bild beeinträchtigen kann.

Um diese potentiellen Fehlerquellen wenigstens zu reduzieren, werden die beiden Bilder kalibriert, also entzerrt und rektifiziert. Hierfür werden vorab in einem Produktionsschritt des Kamerasystems Bilder von einem bekannten Muster, beispielsweise von einem Schachbrett, aufgenommen. Aus diesen Bildern werden verschiedene Parameter, wie Verzeichnungsparamter, Brennweiten, Hauptpunkte des Kamerasystems, sowie Rotation und Translation zwischen den beiden Kamerasensoren ermittelt. Basierend auf diesen im Vorfeld ermitteltet Parametern, können die Bilder nun kalibriert werden.

Abbildungen 7c, 7d zeigen das linke und rechte Bild von Abbildungen 7a, 7b nach erfolgreicher Kalibrierung; die Abbildungen zeigen also als entzerrtes und rektifiziertes rechtes und linkes Bild. Entsprechend sind die Linien des karierten Hintergrunds in diesen Bildern parallel; ein Objektpunkt in beiden Bildern wird nun auf die gleiche Pixelreihe projiziert.

Basierend auf den entzerrten und rektifizierten Bildern wird eine Disparitätskarte zwischen linkem und rechtem Bild sowie eine Disparitätskarte zwischen rechtem und linkem Bild berechnet. Disparitätskarten (oder Tiefenbilder), geben für jeden Bildpunkt dessen Disparität an. Disparitätskarten werden üblicherweise als Farbstufenbilder oder Graustufenbilder codiert, wobei der jeweilige Farb- oder Grauwert die Disparität widerspiegelt. Für die Erstellung der Disparitätskarten werden korrespondierende Pixel zwischen dem linken und rechten Bild gesucht. Bei der Korrespondenzsuche ordnet man Pixel im linken Bild den korrespondierenden Pixeln im rechten Bild zu und umgekehrt. Die zugeordneten Bildelemente sollten das Abbild des gleichen 3-D-Punktes einer Szene sein. Die Verschiebung der Lage aufgrund der unterschiedlichen Perspektiven bezeichnet man als binokulare Disparität und lässt Rückschlüsse auf die Tiefe eines Pixels zu.

Zur Erstellung von Disparitätskarten können - insbesondere in Abhängigkeit von den Erfordernissen (zum Beispiel Qualität, Zeit, etc.) und zur Verfügung stehenden Ressourcen verschiedene Verfahren angewandt werden. Im Kontext der vorliegenden Erfindung hat sich der Semi-Global-Matching Algorithmus nach H.Hirschmüller et al. bewährt, wobei auch andere, dem Fachmann bekannte Verfahren möglich sind.

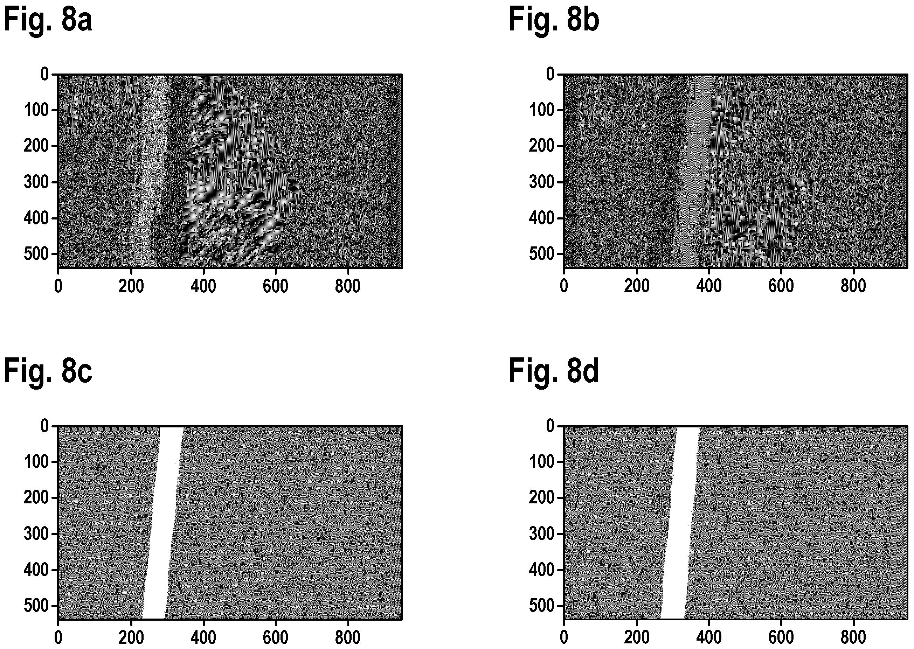

Abbildungen 8a, 8b zeigen die Disparitätskarten basierend auf dem linken und rechten Bild, wobei die Disparität in diesem Fall als Graustufen codiert ist; Pixel mit gleichem Abstand von der Kamera haben also einen entsprechenden Grauwert.

Abbildungen 8c, 8d zeigen die Segmentierung des Instrumentenschafts in der linken und rechten Disparitätskarte. Die Segmentierung erfolgt mittels eines künstlichen Intelligenz- oder computer vision Algorithmus in linkem und rechtem Bild. Hierzu kann beispielsweise eine U-Net Architektur oder ein Liniendetektor verwendet werden. Vorteilhaft ist es, die Segmentierung direkt auf den entzerrten und rektifizierten Bildern durchzuführen. Nachfolgend können in den Disparitätskarten nicht-zugeordnete Bereiche, die der Größe und Form des segmentierten Instruments entsprechen, identifiziert werden. Dies kann beispielsweise zeilenweise durchgeführt werden. Die erkannten Bereiche zeigen den Hintergrund, der durch das Instrument verdeckt wird, sodass keine Stereo-Korrespondenz errechnet werden kann. Die somit ermittelten Hintergrundbereiche können ins jeweilig andere entzerrte und rektifizierte Bild an die Stelle des segmentierten Instrumentenschafts übertragen und somit der Instrumentenschaft überblendet werden.

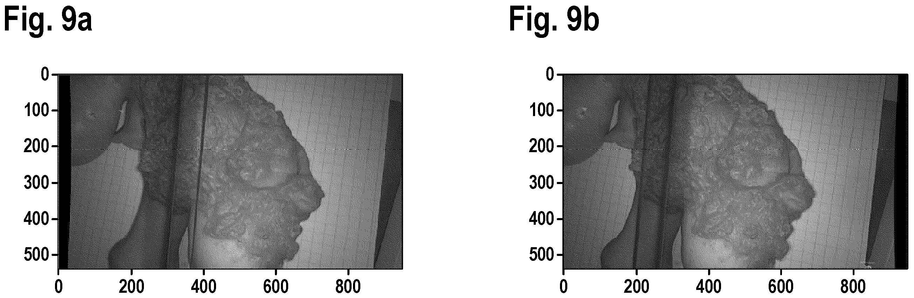

Abbildungen 9a, 9b zeigen die Überlagerung der korrespondierenden Bildausschnitte auf den Instrumentenschaft basierend auf den kalibrierten Bildern. Diese Bilder können anschließend wieder in die ursprüngliche Bilddarstellungsweise (vor der Kalibrierung, mit Verzerrung, etc.) transformiert und dann, je nach Anzeigemodus, in einem Stereobild oder Monobild für den Benutzer dargestellt werden.

Der Umfang dieser Offenbarung schließt alle Änderungen, Ersetzungen, Variationen, Abwandlungen und Modifikationen an den hierin beschriebenen oder veranschaulichten Ausführungsbeispielen ein, die ein durchschnittlicher Fachmann begreifen würde. Der Schutzumfang dieser Offenbarung ist nicht auf die hierin beschriebenen oder veranschaulichten Ausführungsbeispiele beschränkt. Obwohl diese Offenbarung die jeweiligen Ausführungsformen hierin als bestimmte Komponenten, Elemente, Merkmale, Funktionen, Operationen oder Schritte umfassend beschreibt und veranschaulicht, können zudem beliebige dieser Ausführungsformen beliebige Kombinationen oder Permutationen beliebiger Komponenten, Elemente, Merkmale, Funktionen, Operationen oder Schritte, die irgendwo hierin beschrieben oder veranschaulicht sind, umfassen, die ein durchschnittlicher Fachmann begreifen würde. Ein Verweis in den angehängten Patentansprüchen darauf, dass ein Verfahren oder eine Vorrichtung oder eine Komponente einer Vorrichtung oder ein System zum Durchführen einer bestimmten Funktion angepasst, eingerichtet, fähig, konfiguriert, befähigt, betriebsfähig oder betriebsbereit ist, schließt des Weiteren dieses Gerät, dieses System oder diese Komponente ein, unabhängig davon, ob es/sie oder diese bestimmte Funktion aktiviert, eingeschaltet oder freigegeben ist, solange dieses Gerät, dieses System oder diese Komponente dazu angepasst, eingerichtet, fähig, konfiguriert, befähigt, betriebsfähig oder betriebsbereit ist. Obwohl diese Offenbarung bestimmte Ausführungsformen als bestimmte Vorteile bereitstellend beschreibt oder veranschaulicht, können zudem bestimmte Ausführungsformen keine, einige oder alle diese Vorteile bereitstellen.

## Patentansprüche

1. Verfahren zur wenigstens teilweisen optischen Überlagerung von wenigstens einer Information im Vordergrund eines digitalen Bildes, umfassend:
das Aufnehmen, mit einer medizinischen Bildgebungsvorrichtung, wenigstens eines digitalen Bildes in einem Blickfeld der medizinischen Bildgebungsvorrichtung;
das Segmentieren, durch eine Bildbearbeitungsvorrichtung, wenigstens eines Bildbereichs des wenigstens eines Bildes;
das Auswählen, durch die Bildverarbeitungsvorrichtung, des wenigstens einen Bildbereichs in dem wenigstens einem digitalen Bild, welcher der wenigstens eine Information entspricht;
das Überlagern, durch die Bildbearbeitungsvorrichtung, des Bildbereichs in dem wenigstens einem Bild mit Bildinformation; und
und das Darstellen, auf einer Darstellungsvorrichtung, wenigstens eines Bildes zusammen mit dem überlagerten Bildbereich;
**dadurch gekennzeichnet, dass** die Bildinformation dem, im Blickfeld der medizinischen Bildgebungsvorrichtung durch die wenigstens eine Information verdecktem Bereich entspricht.

2. Verfahren nach Anspruch 1, wobei die medizinischen Bildgebungsvorrichtung eine Endoskopkamera oder eine Exoskopkamera ist.

3. Verfahren nach Anspruch 1 oder 2, wobei die Bildinformation aus wenigstens einem Bild extrahiert werden, dass zeitlich vor dem wenigstens ein digitales Bild aufgenommen wurde und in dem die Bildinformation, die dem in dem wenigstens einem digitalen Bild durch die wenigstens eine Information verdecktem Bereich entspricht, enthalten ist.

4. Verfahren nach Anspruch 1 oder 2, wobei die Bildinformation unter Zuhilfenahme von auf künstlicher Intelligenz basierender generative Bildbearbeitungsmethoden durch die Bildbearbeitungsvorrichtung erzeugt wird.

5. Verfahren nach Anspruch 1 oder 2, wobei die Bildinformation aus wenigstens einem, aus einer anderen Perspektive aufgenommen Bild extrahiert wird.

6. Verfahren nach Anspruch 5, wobei das wenigstens eine aus einer anderen Perspektive aufgenommen Bild mit einer zweiten medizinischen Bildgebungsvorrichtung aufgenommen wird.

7. Verfahren nach Anspruch 5, wobei das aus einer anderen Perspektive aufgenommen Bild nach Bewegung der medizinischen Bildgebungsvorrichtung aufgenommen wird.

8. Verfahren nach Anspruch 7, wobei die Bewegung der medizinischen Bildgebungsvorrichtung durch eine motorisierte Steuerung erfolgt.

9. Verfahren nach Anspruch 7 oder 8, wobei die Bewegung senkrecht zur Hauptausrichtung der Information erfolgt.

10. Verfahren nach Anspruch 5, wobei die medizinischen Bildgebungsvorrichtung zwei nebeneinander angeordnete Objektivlinsen umfasst mit der jeweils ein rechtes und ein linkes Bildaufgenommen wird, das wenigstens eine digitale Bild ein dreidimensionales Bild ist und die Bildinformation als zweidimensionale Information aus dem linken Bild und/oder dem rechten Bild extrahiert wird.

11. Verfahren nach Anspruch 10, wobei Bildinformation, die nicht aus dem linken Bild und/oder dem rechten Bild extrahiert werden kann, gemäß der Verfahren aus Anspruch 3 extrahiert und/oder aus Anspruch 4 generiert wird.

12. Verfahren nach einem der vorstehenden Ansprüche, wobei der überlagerte Bildbereich als transparent, teil-transparent und/oder als Umriss dargestellt wird.

13. Verfahren nach Anspruch 12, wobei Art der Darstellung des überlagerten Bildbereichs abhängig von der Zuverlässigkeit der verwendeten Bildinformation ist.

14. Verfahren nach Anspruch 13, wobei die Zuverlässigkeit der Verwendeten Bildinformation über eine Plausibilitätsprüfung basierend auf vordefinierter Metriken bestimmt wird.

15. Verfahren nach einem der vorstehenden Ansprüche, wobei die Zuverlässigkeit der Überlagerung durch Vergleich der überlagerten Bildinformation mit dem wenigstens einem Bild bestimmt wird.

16. System umfassend wenigstens eine medizinischen Bildgebungsvorrichtung, wenigstens eine Bildbearbeitungsvorrichtung und wenigstens eine Darstellungsvorrichtung, wobei das System konfiguriert ist ein Verfahren gemäß einem der Ansprüche 1 bis 15 auszuführen.

17. System nach Anspruch 16, wobei die Bildbearbeitungsvorrichtung einen Diskriminator ausführt, mit dem die Zuverlässigkeit, der durch auf künstliche Intelligenz basierenden generativen Bildbearbeitungsmethoden generierten Bildinformationen bestimmt wird.

18. Computerprogramm, umfassend Befehle, die bei der Ausführung des Programms durch einen Computer diesen veranlassen, die Verfahren gemäß einem der Ansprüche 1 bis 15 auszuführen.

19. Computerlesbares Speichermedium, umfassend Befehle, die bei der Ausführung durch einen Computer diesen veranlassen, die Verfahren gemäß einem der Ansprüche 1 bis 15 auszuführen.
